# EUROPEAN PATENT SPECIFICATION

(11) **EP 3 239 275 B1**
(45) Date of publication and mention of the grant of the patent: **06.05.2020**
(21) Application number: 15872331.2
(22) Date of filing: 10.06.2015
(51) Int. Cl.: C10G 1/00, C07C 1/02, C07B 61/00, C10G 2/00, C07B 31/00

(54) **METHOD FOR SYNTHESIZING HYDROCARBON**
VERFAHREN ZUR SYNTHESE VON KOHLENWASSERSTOFF
PROCÉDÉ POUR LA SYNTHÈSE D'HYDROCARBURE

(30) Priority: 25.12.2014 JP 2014261441
(43) Date of publication of application: 01.11.2017
(73) Proprietor: SI Energy Company Limited, Osaka-shi Osaka 5410051 (JP)
(72) Inventor: IMANAKA, Tadayuki, Osaka 565-0873 (JP); TAKEMOTO, Tadashi, Okayama 708-0001 (JP)
(74) Representative: Van Breda, Jacobus
(86) International application number: PCT/JP2015/066657
(87) International publication number: WO 2016/103762

(56) References cited:
- WO-A1-2014/170200
- JP-A- S55 105 625
- JP-A- 2000 335 901
- JP-A- 2010 089 055
- JP-A- 2012 115 750
- JP-B2- 5 131 444
- US-A1- 2010 213 046
- US-A1- 2012 315 215
- US-A1- 2013 239 469

## Description

### Field of the Invention

The present invention relates to a method for synthesizing a hydrocarbon by reducing carbon dioxide in water.

### Background of the Invention

As a method for synthesizing a hydrocarbon by reducing carbon dioxide in water, there has hitherto been known a method in which the synthesis is performed by adding hydrogen under conditions of high temperature and high pressure. However, such a conventional method performs the synthesis by adding hydrogen under the conditions of high temperature and high pressure, and accordingly is unfortunately high in the apparatus cost, and makes cumbersome the apparatus maintenance.

Consequently, there has been proposed a method for synthesizing a hydrocarbon without requiring the addition of hydrogen and the conditions of high temperature and high pressure (for example, see Japanese Patent No. 5131444). In the method for synthesizing a hydrocarbon shown in Japanese Patent No. 5131444, a gas column of carbon dioxide is formed in water, a swirling flow of water is generated around the gas column, thus carbon dioxide is fed into water as fine gas bubbles, the water containing fine gas bubbles of carbon dioxide is irradiated with ultraviolet light in the presence of a photocatalyst in the atmospheric pressure atmosphere to reduce the carbon dioxide, and thus a hydrocarbon is synthesized.

However, in the method for synthesizing a hydrocarbon as shown in Japanese Patent No. 5131444, the formation of a gas column of carbon dioxide in water is always required, and the generation of a swirling flow of water around the gas column of carbon dioxide is also required; thus, a mechanism for forming the gas column of carbon dioxide and the swirling flow of water is required, and thus, the reaction mechanism is unfortunately complicated.

Accordingly, an object of the present invention is to provide a method for synthesizing a hydrocarbon, capable of efficiently synthesizing a hydrocarbon by reducing carbon dioxide in water on the basis of an easy reaction mechanism, and an apparatus for synthesizing a hydrocarbon.

JP 2012 115 750 discloses an apparatus suitable for reducing carbon dioxide in water comprising a bubble generation unit suitable for feeding oxygen into water, and a unit for irradiating the water with ultraviolet radiation.

### Disclosure of the Invention

The invention is a method for synthesizing a hydrocarbon by reducing carbon dioxide in water, wherein nanobubbles of oxygen are generated by feeding oxygen into water containing carbon dioxide, water containing the nanobubbles of oxygen is irradiated with ultraviolet light in the presence of a photocatalyst to produce active oxygen, and carbon dioxide is reduced in the presence of the active oxygen.

Also described is a method wherein carbon dioxide is reduced in the presence of a separately prepared liquid hydrocarbon and the active oxygen produced from the nanobubbles of oxygen.

According to the present invention, a hydrocarbon is synthesized by reducing carbon dioxide in the presence of active oxygen produced by irradiating water containing nanobubbles of oxygen with ultraviolet light, and accordingly, a hydrocarbon can be synthesized simply by using water containing carbon dioxide. Accordingly, a hydrocarbon can be synthesized with an easy reaction mechanism, and at the same time, a hydrocarbon can be synthesized efficiently.

In addition, but not according to the present invention, carbon dioxide is reduced in the presence of a separately prepared liquid hydrocarbon and the active oxygen produced from the nanobubbles of oxygen, a hydrocarbon can be synthesized in a larger amount.

### Brief Description of the Drawings

FIG. 1 is a schematic diagram illustrating an outline of a configuration of an embodiment of a synthesis apparatus for synthesizing a hydrocarbon by a method for synthesizing a hydrocarbon according to the present invention; and

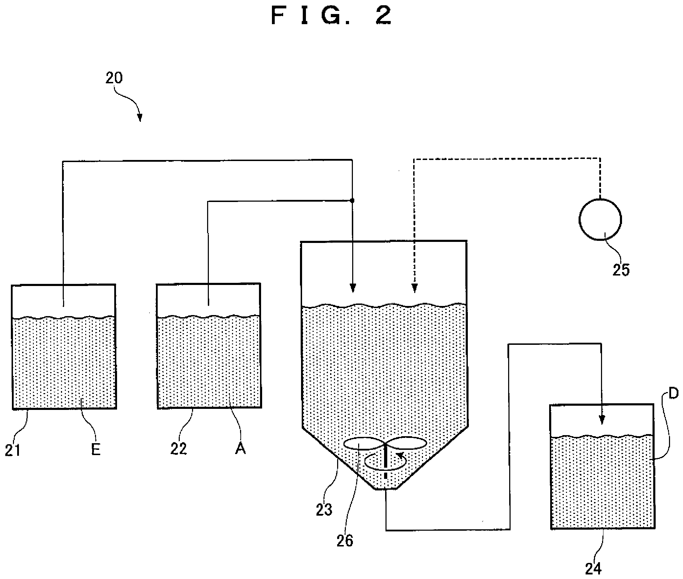

FIG. 2 is a schematic diagram illustrating an outline of a configuration of another synthesis apparatus for synthesizing a hydrocarbon by a method for synthesizing a hydrocarbon not according to the present invention.

### Description of the Embodiments

First, the method for synthesizing a hydrocarbon according to the present invention (the first method), and the synthesis apparatus of the first method.

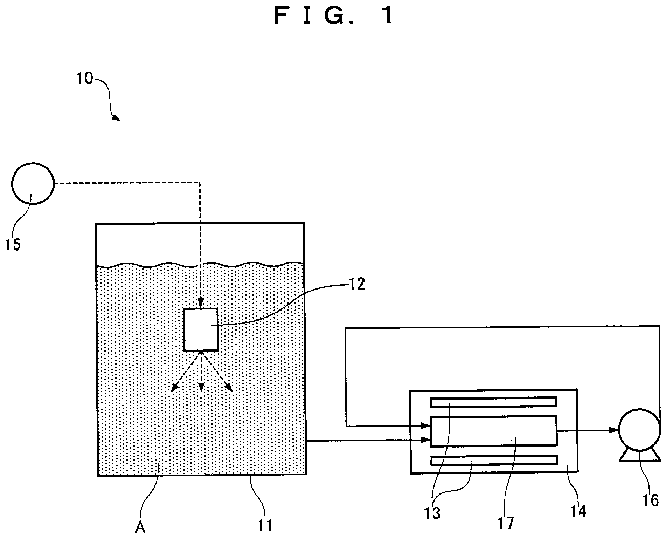

As shown in FIG. 1, the synthesis apparatus 10 for synthesizing a hydrocarbon by the method for synthesizing a hydrocarbon according to the present invention includes: a water tank 11 for containing carbon dioxide-dissolved water A; a nanobubble generator 12 (an example of "the nanobubble generation unit") for generating nanobubbles of oxygen (ultrafine bubbles of oxygen of a few hundred nanometers or less); and a photocatalyst apparatus 14 (an example of "the ultraviolet light irradiation unit") for irradiating the water A containing nanobubbles of oxygen with ultraviolet light in the presence of a photocatalyst (such as titanium oxide or zinc oxide).

In the water tank 11, a predetermined amount of water A allowed to pass through a reverse osmosis membrane is contained. In the water A contained in the water tank 11, carbon dioxide is dissolved. It is to be noted that although not shown in FIG. 1, a carbon dioxide feed source such as a carbon dioxide cylinder is provided outside the water tank 11, and there may be adopted a configuration in which carbon dioxide is fed from the aforementioned carbon dioxide feed source to the water tank 11 (a configuration to fill the interior of the water tank 11 with carbon dioxide). The water A is not limited to water allowed to pass through a reverse osmosis membrane, but any carbon dioxide-dissolving water may be adopted. The water A is preferably a water allowed to pass through a reverse osmosis membrane to remove impurities such as ions or salts.

The nanobubble generator 12 is an ultrafine pore type nanobubble generator. The nanobubble generator 12 is connected to an oxygen feed source 15 such as an oxygen cylinder, and generates nanobubbles of oxygen in the interior of the water tank 11 on the basis of the oxygen fed from the oxygen feed source 15.

The nanobubble generator 12 includes an oxygen jetting section for jetting a gaseous layer (gas bubbles) of oxygen and a water jetting section for jetting the water A in the water tank 11. In the nanobubble generator 12, the oxygen jetting section and the water jetting section are placed in the water tank 11.

In the oxygen jetting section, a special ceramic filter having nano-level fine pores is arranged, and from the aforementioned fine pores, a gaseous layer (gas bubbles) of oxygen is jetted. In the water jetting section, the water A in the water tank 11 is jetted to the special ceramic filter, and consequently the liquid flow of the water A flows on the surface of the special ceramic filter.

In the nanobubble generator 12, by giving the liquid flow of the water A in the water tank 11 to the boundaries of the fine pores of the special ceramic filter, the gaseous layer (gas bubbles) of oxygen jetted from the oxygen jetting section (fine pores) is finely cut. Then, the cut gaseous layer (gas bubbles) of oxygen is compressed by the surface tension of the water A in the water tank 11, and thus nanobubbles (ultrafine gas bubbles) of oxygen are generated. It is to be noted that the nanobubble generator 12 is not limited to an ultrafine pore type, and may be any other heretofore known nanobubble generator that is an apparatus capable of generating nanobubbles of oxygen.

As shown in FIG. 1, the photocatalyst apparatus 14 has UV lamps 13 for irradiating the water A containing nanobubbles of oxygen with ultraviolet light, and a reaction tube 17 provided with a photocatalyst in the interior thereof. The UV lamps 13 are arranged around the reaction tube 17, and radiate ultraviolet light to the reaction tube 17. The reaction tube 17 is a tubular vessel capable of transmitting ultraviolet light, and is constituted so as to allow the water A containing nanobubbles of oxygen to pass through the inside thereof.

In the photocatalyst apparatus 14, the water A containing nanobubbles of oxygen is fed at a predetermined flow rate in the inside of the reaction tube 17 charged with a photocatalyst, and the aforementioned water A passing through the inside of the reaction tube 17 is irradiated with ultraviolet light. Then, the water A having passed through the photocatalyst apparatus 14 is again got back to the photocatalyst apparatus 14 by a circulation pump 16, and is circulated for a predetermined time by the circulation pump 16.

In the synthesis apparatus 10, first, nanobubbles of oxygen are generated by the nanobubble generator 12 in the water A containing carbon dioxide in the water tank 11. In this way, the generated nanobubbles of oxygen stay in the water A in the water tank 11 (visually transparent). Then, the water A containing the generated nanobubbles of oxygen is fed to the photocatalyst apparatus 14, and thus, the water A containing the nanobubbles of oxygen is irradiated with ultraviolet light in the presence of a photocatalyst. In this way, as shown in the reaction formula (1), the active oxygen such as a superoxide anion radical or a hydroxyl radical is produced from oxygen in a nanobubble state through the intermediary of ozone.

302 → 2O₃ → active oxygen (O₂⁻·, OH· or the like) (1)

At the same time, as shown in the reaction formula (2), the reduction reaction of the carbon dioxide dissolved in the water A occurs.

CO₂ + H₂O → CO + H₂ + O₂ (2)

The reduction reaction of carbon dioxide in the reaction formula (2) occurs in the presence of the active oxygen produced in the reaction formula (1), and accordingly, the reaction shown in the reaction formula (3) proceeds. By the reaction shown in the reaction formula (3), a hydrocarbon is synthesized.

(2n+1) H₂ + nCO → CₙH₂ₙ₊₂ + nH₂O (3)

In other words, a hydrocarbon is synthesized by reducing carbon dioxide in the presence of the active oxygen produced from the oxygen in a nanobubble state.

As described above, the synthesis apparatus 10 has a constitution such that nanobubbles of oxygen are generated in the water A containing carbon dioxide dissolved therein, and a hydrocarbon is synthesized by reducing carbon dioxide by irradiating the water A with ultraviolet light in the photocatalyst apparatus 14 while the water A containing the aforementioned nanobubbles of oxygen is being circulated; consequently, a hydrocarbon can be synthesized simply by using water containing carbon dioxide and nanobubbles of oxygen (without forming a gas column of carbon dioxide or a swirling flow of water). Accordingly, a hydrocarbon can be synthesized on the basis of a facile reaction mechanism, and a hydrocarbon can also be synthesized efficiently.

Next, another synthesis method (the second method) of the method for synthesizing a hydrocarbon not according to the present invention and the synthesis apparatus of the another synthesis method are described.

The another method of the method for synthesizing a hydrocarbon according to the present invention is a method for newly synthesizing a liquid hydrocarbon by reducing carbon dioxide in the presence of a separately prepared liquid hydrocarbon and the active oxygen produced by the above-described synthesis method.

Herein, the separately prepared liquid hydrocarbon means a liquid hydrocarbon preliminarily prepared by a method other than the aforementioned second method, and being a liquid hydrocarbon (source oil) having an approximately the same composition as the composition of the liquid hydrocarbon to be synthesized by the second method. In other words, the separately prepared liquid hydrocarbon means a liquid hydrocarbon (source oil) preliminarily prepared by a different method other than the above-described first method and the aforementioned second method concerned. In the case where a liquid hydrocarbon is preliminarily synthesized by the above-described first method, the resulting liquid hydrocarbon is also included in the separately prepared liquid hydrocarbon. Moreover, examples of the separately prepared liquid hydrocarbon (source oil) include a hydrocarbon having 6 to 36 carbon atoms such as light oil and kerosene.

The synthesis apparatus 20 for synthesizing a hydrocarbon by this method (the second method) include: a first feed tank 21 for feeding the separately prepared liquid hydrocarbon E (source oil); a second feed tank 22 for feeding the water A containing the active oxygen produced by the above-described first method; a reaction tank 23 for allowing the liquid hydrocarbon E and the water A containing active oxygen to react with each other; and a still standing tank 24 for allowing the liquid hydrocarbon E (new oil) after the reaction and the water A to stand still.

In the synthesis apparatus 20, first, a liquid mixture composed of the separately prepared liquid hydrocarbon E (source oil) and the water A containing the active oxygen produced by the above-described first method is fed to the reaction tank 23 while the liquid mixture is being sprayed under a predetermined pressure. In this way, micelles are formed between the liquid hydrocarbon E and the water A containing active oxygen. At the same time, the interior of the reaction tank 23 is filled with carbon dioxide by feeding carbon dioxide from a carbon dioxide feed source 25 such as a carbon dioxide cylinder to the reaction tank 23. Herewith, carbon dioxide is taken into the micelles formed as described above. Simultaneously, in the reaction tank 23 filled with carbon dioxide, the liquid hydrocarbon E and the water A containing the active oxygen are stirred by a stirrer 26 of the reaction tank 23. It is to be noted that the temperature inside the reaction tank 23 is from room temperature to preferably approximately 40°C and more preferably to approximately 30°C. In addition, the pressure inside the reaction tank 23 is the atmospheric pressure.

After the stirring (after the reaction), the liquid mixture D composed of the liquid hydrocarbon E and the water A is fed from the reaction tank 23 to the still standing tank 24. Then, the aforementioned liquid mixture D is allowed to stand still for a predetermined time (for example, 24 hours). Herewith, the liquid hydrocarbon E is produced as a supernatant liquid of the liquid mixture D in the still standing tank 24 in the upper layer of the liquid mixture D. The amount of the liquid hydrocarbon E (new oil) produced in the upper layer of the liquid mixture D is increased by 10 to 15% as compared with the amount of the separately prepared liquid hydrocarbon E (source oil). In other words, a new liquid hydrocarbon E (new oil) is produced by the second method.

Alternatively, it is also possible to repeat the second method by isolating the liquid hydrocarbon E (new oil) produced in the upper layer of the liquid mixture D from the liquid mixture D, mixing the isolated liquid hydrocarbon E (new oil) with the water A containing the active oxygen, and again feeding the resultant mixture to the reaction tank 23. In this way, the amount of the liquid hydrocarbon E (new oil) produced in the upper layer of the liquid mixture D is increased by 20 to 30% as compared with the amount of the separately prepared liquid hydrocarbon E (source oil). In other words, by repeating a plurality of times the second method, the amount of the newly produced liquid hydrocarbon E (new oil) is further increased.

In this way, in the synthesis apparatus 20, carbon dioxide can be reduced by mixing the separately prepared liquid hydrocarbon (source oil) and the water containing nanobubbles of oxygen, and accordingly as compared with the case where the separately prepared liquid hydrocarbon (source oil) is not included, the reduction of carbon dioxide is promoted and the hydrocarbon can be synthesized in a larger amount. In other words, by further adding the separately prepared liquid hydrocarbon in the presence of the active oxygen produced by irradiating water containing nanobubbles of oxygen with ultraviolet light, the reduction of carbon dioxide is promoted and the hydrocarbon is efficiently synthesized.

Hereinafter, Example 1 of the present invention and Comparative Example 1 and Comparative Example 2 in relation to Example 1 are described. It is to be noted that the present invention is not limited to Example 1 at all.

### Example 1

In the synthesis apparatus 10, 50 L of water obtained by allowing tap water to pass through a reverse osmosis membrane was placed in the water tank 11. Then, the nanobubble generator 12 was operated in the water tank 11 to jet nanobubbles of oxygen into the aforementioned water, and carbon dioxide was jetted into the aforementioned water from a carbon dioxide cylinder arranged outside the water tank 11.

While the water into which nanobubbles of oxygen and carbon dioxide were jetted was being fed at a flow rate of 18 L/min to the photocatalyst apparatus 14, the water was irradiated with ultraviolet light by using the UV lamps 13 in the presence of titanium oxide (photocatalyst). The aforementioned water was circulated between the photocatalyst apparatus 14 and the water tank 11 for 24 hours.

It is to be noted that in order to allow nanobubbles of oxygen and carbon dioxide to stay (to be dissolved) sufficiently in the water tank 11, nanobubbles of oxygen and carbon dioxide were continuously jetted into the water tank 11 to be dissolved in the water even while the water was circulated between the photocatalyst apparatus 14 and the water tank 11 for 24 hours. In order to prevent the volatilization of the produced hydrocarbon, the upper surface of the water tank 11 was sealed with a seal material.

### Comparative Example 1

In the synthesis apparatus 10, 50 L of water obtained by allowing tap water to pass through a reverse osmosis membrane was placed in the water tank 11. Then, oxygen was fed into the water tank 11 from an oxygen cylinder arranged outside the water tank 11 to jet oxygen into the aforementioned water, and carbon dioxide was jetted into the aforementioned water from a carbon dioxide cylinder arranged outside the water tank 11. In other words, oxygen not being in a state of nanobubbles was fed to the water.

Moreover, while the water into which oxygen and carbon dioxide were jetted was being fed at a flow rate of 18 L/min to the photocatalyst apparatus 14, the water was irradiated with ultraviolet light by using the UV lamps 13 in the presence of titanium oxide (photocatalyst). The aforementioned water was circulated between the photocatalyst apparatus 14 and the water tank 11 for 24 hours.

It is to be noted that similarly to Example 1, in order to allow oxygen and carbon dioxide to stay (to be dissolved) sufficiently in the water tank 11, oxygen and carbon dioxide were continuously jetted into the water tank 11 to be dissolved in the water even while the water was circulated between the photocatalyst apparatus 14 and the water tank 11 for 24 hours. In order to prevent the volatilization of the produced hydrocarbon, the upper surface of the water tank 11 was sealed with a seal material.

### Comparative Example 2

In the synthesis apparatus 10, 50 L of water obtained by allowing tap water to pass through a reverse osmosis membrane was placed in the water tank 11. Then, while the aforementioned water was being fed at a flow rate of 18 L/min to the photocatalyst apparatus 14, the water was irradiated with ultraviolet light by using the UV lamps 13 in the presence of titanium oxide (photocatalyst). Then, the aforementioned water was circulated between the photocatalyst apparatus 14 and the water tank 11 for 24 hours. In other words, in Comparative Example 2, only the dissolved oxygen and the dissolved carbon dioxide being dissolved in the water placed in the water tank 11 were used, and the amounts of oxygen and carbon dioxide fed to the water were made smaller as compared with Example 1 and Comparative Example 1. In order to prevent the volatilization of the produced hydrocarbon, the upper surface of the water tank 11 was sealed with a seal material.

In each of Example 1, Comparative Example 1 and Comparative Example 2, a certain amount of water was sampled from the water circulated between the photocatalyst apparatus 14 and the water tank 11 for 24 hours, and from the sampled water, a hydrocarbon was extracted by using diethyl ether. Then, the extracted hydrocarbon was completely dehydrated, and then analyzed with a GC-Mass (SHIMADZU GC-2010).

As a result of performing the analysis with the GC-Mass, the hydrocarbons extracted in Example 1, Comparative Example 1 and Comparative Example 2 were found to be saturated hydrocarbons having 15 to 20 carbon atoms.

As a result of measuring the amounts of the saturated hydrocarbons produced in Example 1, Comparative Example 1 and Comparative Example 2, it was verified that 500 mg of a saturated hydrocarbon, 200 mg of a saturated hydrocarbon and 100 mg or less of a saturated hydrocarbon were produced in Example 1, Comparative Example 1 and Comparative Example 2, respectively. In other words, it has been found that a saturated hydrocarbon is produced in a high yield by treating water containing nanobubbles of oxygen in the photocatalyst apparatus 14. It has also been found that in order to produce a saturated hydrocarbon in a high yield, it is necessary to feed sufficient amounts of oxygen and carbon dioxide to the water to be treated.

Next, Example 2 of the present invention and Comparative Example 3 in relation to Example 2 are described. It is to be noted that the present invention is not limited by Example 2 at all.

### Example 2 (not according to the invention)

In the synthesis apparatus 10, 100 L of water obtained by allowing tap water to pass through a reverse osmosis membrane was placed in the water tank 11. Then, the nanobubble generator 12 was operated for 120 minutes in the water tank 11 to jet nanobubbles of oxygen into the water and the nanobubbles of oxygen were retained in the water.

Moreover, while the water containing nanobubbles of oxygen was being fed at a flow rate of 18 L/min to the photocatalyst apparatus 14, the water was irradiated with ultraviolet light by using the UV lamps 13 in the presence of titanium oxide (photocatalyst). Then, the water containing nanobubbles of oxygen was circulated in the photocatalyst apparatus 14 for 30 minutes.

Moreover, a liquid mixture composed of 2.5 1 of a preliminarily prepared light oil (source oil) and 2.5 L of the water containing nanobubbles of oxygen treated in the photocatalyst apparatus 14 was fed to the reaction tank 23 while the liquid mixture was being sprayed under a pressure of 1.0 MPa. Simultaneously, 500 L or more of carbon dioxide was fed under a pressure of 0.3 MPa to the reaction tank 23 to fill the reaction tank 23 with carbon dioxide. Simultaneously, the light oil and the water were stirred for 4 minutes in the reaction tank 23 filled with carbon dioxide. It is to be noted that the temperature in the reaction tank 23 was set at 30°C. The reaction was performed in the atmospheric pressure atmosphere.

After the stirring for 4 minutes (after the reaction), the liquid mixture composed of the light oil and the water was fed from the reaction tank 23 to the still standing tank 24, and was allowed to stand still in the still standing tank 24 for 24 hours. The temperature inside the still standing tank 24 was set at 35°C. The still standing of the liquid mixture was performed in the atmospheric pressure atmosphere.

### Comparative Example 3

In Comparative Example 3, the treatment was performed under the same conditions as in Example 2 except that the oxygen to be fed to the water placed in the water tank 11 was altered from "the nanobubbles of oxygen" in foregoing Example 2 to "the oxygen not being in a state of nanobubbles" jetted from the oxygen cylinder arranged outside the water tank 11 (the state in which the oxygen fed from the oxygen cylinder was directly jetted into the water tank 11).

In Example 2, after the still standing for 24 hours, the supernatant liquid was isolated from the aforementioned liquid mixture in the still standing tank 24, and the isolated supernatant liquid (new oil) was analyzed. The analysis was performed with respect to the items shown in Table 1. As a comparison, the light oil (source oil) before the treatment in the reaction tank 23 was also analyzed with respect to the same items. Consequently, as shown in Table 1, the supernatant liquid (new oil) was found to be a light oil comparable to the light oil (source oil) before the treatment in the reaction tank 23.

**[Table 1]**

| Items | Units | Results | | Test methods |
|---|---|---|---|---|
| | | Source oil | New oil | |
| 1. Reaction | - | Neutral | Neutral | JIS K2252 |
| 2. Flash point (PMCC) | °C | 73.0 | 82.0 | JIS K2265-3 |
| 3. Kinematic viscosity (30°C) | mm²/s | 3.479 | 3.710 | JIS K2283 |
| 4. Pour point | °C | -15.0 | -12.5 | JIS K2269 |
| 5. Carbon residue content of 10% residual oil | Mass fraction % | 0.01 | 0.04 | JIS K2270-2 |
| 6. Moisture content Karl Fisher method | Mass fraction % | 0.0063 | 0.010 | JIS K2275 |
| 7. Ash content | Mass fraction % | 0.001 | 0.001 | JIS K2272 |
| 8. Sulfur content | Mass fraction % | 0.0007 | 0.0007 | JIS K2541-6 |
| 9. Density (15°C) | g/cm³ | 0.8295 | 0.8311 | JIS K2249-1 |
| 10. Distillation characteristic | | | | JIS K2254 |
| 10% Distillation temperature | °C | 217.0 | 226.0 | |
| 50% Distillation temperature | °C | 271.5 | 274.5 | |
| 90% Distillation temperature | °C | 326.0 | 328.5 | |
| 11. Cetane index | - | 56.2 | 56.9 | JIS K2280-5 |
| 12. Gross calorific value | J/g | 45990 | 46010 | JIS K2279 |
| 13. Plugging point | - | -10 | -10 | JIS K2269 |

In each of Example 2 and Comparative Example 3, the amount of the supernatant liquid (light oil) isolated from the aforementioned liquid mixture in the still standing tank 24 was measured. Consequently, in Example 2, the amount of the supernatant liquid (light oil) was 2.80 L. Specifically, the amount of the preliminarily prepared light oil was 2.5 L, and hence the amount of the newly synthesized light oil was found to be 0.3 L (yield: 12%). On the other hand, in Comparative Example 3, the amount of the supernatant liquid (light oil) was 2.58 L. Specifically, the amount of the newly synthesized light oil was found to be 0.08 L (yield: 3.2%). From the above-described results, it has been able to be verified that the use of "the nanobubbles of oxygen" increases the amount (yield) of the newly synthesized light oil.

## Claims

1. A method for synthesizing a hydrocarbon by reducing carbon dioxide in water,
wherein nanobubbles of oxygen are generated by feeding oxygen into water containing carbon dioxide;
water containing the nanobubbles of oxygen is irradiated with ultraviolet light in the presence of a photocatalyst to produce active oxygen; and
carbon dioxide is reduced in the presence of the active oxygen.

## Patentansprüche

1. Ein Verfahren zur Synthese eines Hydrocarbons durch Reduktion von Kohlendioxid in Wasser,
wobei Nanobläschen aus Sauerstoff dadurch erzeugt werden, dass Sauerstoff in Kohlendioxid enthaltendes Wasser eingeleitet wird;
wobei Wasser, welches die Nanobläschen aus Sauerstoff enthält, mit ultraviolettem Licht im Beisein von einem Photokatalysator bestrahlt wird, um aktiven Sauerstoff zu erzeugen; und
wobei Kohlendioxid im Beisein des aktiven Sauerstoffs reduziert wird.

## Revendications

1. Procédé pour synthétiser un hydrocarbure par réduction de dioxyde de carbone dans de l'eau,
dans lequel des nanobulles d'oxygène sont générées par introduction d'oxygène dans de l'eau contenant du dioxyde de carbone ;
l'eau contenant les nanobulles d'oxygène est soumise à un rayonnement avec une lumière ultraviolette en présence d'un photocatalyseur pour produire de l'oxygène actif ; et
le dioxyde de carbone est réduit en présence de l'oxygène actif.
